# EUROPEAN PATENT SPECIFICATION

(11) **EP 2 516 620 B1**
(45) Date of publication and mention of the grant of the patent: **14.09.2016**
(21) Application number: 10748067.5
(22) Date of filing: 02.07.2010
(51) Int. Cl.: C12M 1/00, C12M 3/08, C12N 5/00

(54) **APPARATUS AND METHOD FOR CELL EXPLOITATION**
VORRICHTUNG UND VERFAHREN FÜR ZELLNUTZUNG
APPAREIL ET PROCÉDÉ D'EXPLOITATION CELLULAIRE

(30) Priority: 23.12.2009 EP 09180667
(43) Date of publication of application: 31.10.2012
(73) Proprietor: Fraunhofer-Gesellschaft zur Förderung der angewandten Forschung e.V., 80686 München (DE)
(72) Inventor: AKRA, Bassil, 82140 Olching (DE)
(74) Representative: Gleiss & Grosse
(86) International application number: PCT/EP2010/059462
(87) International publication number: WO 2011/076447

(56) References cited:
- WO-A1-2008/097155
- US-A- 4 028 190
- US-A1- 2002 132 341
- US-A1- 2004 067 582
- US-A1- 2005 203 636

## Description

The present invention relates to an apparatus and a method for exploitation of cells from a sample, in particular for automated exploitation of cells. In particular, the present invention relates to an apparatus and a method for exploitation and harvesting of cells from a tubular biological sample, like a vessel or an umbilical cord.

### BACKGROUND

The method of tissue engineering enables new possibilities for the generation of artificial tissue for medical applications (Vesely et al., 2005, Dohmen et al., 2006). Due to this fact research has been performed in order to remedy the problems of the currently used prostheses. For this purpose, different materials as well as different sources of cells have been used (Neuenschwander et al., 2004, Mendelson et al., 2006). The cellularization of the matrices has been carried out either *in vivo* or *in vitro.*

Different devices for cell exploitation are know from prior art. US patent application-2002/0132341 A1, for example, discloses an apparatus for the isolation of pancreatic islets. The apparatus comprises a digestion chamber which may be filled with a process solution in which a pancreatic tissue sample may be added. Under operation the sample and the process solution circulate together through the digestion chamber to liberate the islets into the process solution. A further example is provided by US patent application 4,028,190 which discloses a system for tissue cell recovery. The system comprises a processing tank which may be filled with different processing media. A sample, for example shredded embryos, may be added to the processing tank where cells of the sample are liberated into the media within the processing tank. After separation of the cells from the sample the processing tank may be drained through a filter to remove undesirable materials such as undigested tissue.

The document US 2004/0067582 discloses an apparatus and methods for preparing devitalized vein tissue implants by removal of cells in an apparatus with a treatment chamber where the vein is fixed and tubing, valves and pumps for flowing different washing solutions through the vein tissue.

At present, endothelial cells and fibroblasts are exploited particularly from the *vena saphena magna* remaining after aorta coronary bypass operations by a manual method. Vessel cannules are inserted at both ends of the blood vessel and fixed manually with surgical suture material. A washing medium being prewarmed at 37°C is then injected using a 20 ml syringe through a three-way valve mounted at both ends of the blood vessel, wherein the blood vessel can be cleaned and checked for impermeability.

Afterwards, the blood vessel is filled with Trypsin-EDTA solution (10x) or 1% collagenase solution and subsequently incubated in PBS at 37°C and 5% CO₂ for about 25 minutes. The enzymatic reaction is stopped by injecting a stop medium, e.g. M199, into the vessel and the cell suspension is centrifuged at 1000 rpm at 4°C for about 10 minutes, resuspended in a growth medium (i.e. a culture medium for endothelial cells including 10% FCS) and plated in cell culture flasks.

The exploitation of the fibroblasts takes place subsequently, according to the exploitation of the endothelial cells, wherein the incubation time of 2% collagenase solution within the blood vessel is increased up to about 30 minutes, the enzymatic reaction is stopped by a stop medium, the cell suspension is centrifuged at 1000 rpm at 4°C for about 10 minutes, resuspended in a culture medium for fibroblasts including 10% FCS and plated in cell culture flasks.

The method described above provides long duration periods of up to 90 minutes based on the complex handling procedure. Due to the manual and individual handling, the efficiency and purity of cell exploitation highly depends on the routine and experience of the operator which frequently leads to a variety of different and often non-reproducible exploitation results. Further, high loss of cells and unsterility are possible consequences of practising the manual method for cell exploitation.

Therefore there is an unmet need for an improved apparatus and method for exploitation of cells from a sample.

According to the invention this need is settled by-an apparatus and a method for automated exploitation of cells from a sample as defined in the independent claims. Preferred embodiments are subject of the dependent claims.

### SUMMARY OF THE INVENTION

The apparatus according to the invention comprises all the technical features mentioned in claim 1, among them comprises a plurality of dispensing containers for the separated storage of fluid media. Each dispensing container comprises at least one outlet, wherein a media delivery arrangement is preferably associated to each of the dispensing containers.

A controlled dispensing of the fluid from each of the dispensing containers is achieved by at least one first valve which is connected to each of the outlets. Preferably, the number of valves according to the apparatus of the invention corresponds to the number of dispensing containers and each of the first valves is connected to the outlet of one dispensing container and each dispensing container is connected to one of the first valves. According to another aspect, the at least one first valve is preferably a multi-way valve, wherein the number of ways of the multi-way valve preferably corresponds to the number of dispensing containers.

The apparatus according to the invention comprises a plurality of collecting containers for the collection of fluid media, each collecting container comprising an inlet, wherein at least one second valve is connected to the inlet of each collecting container. Again, the number of second valves preferably corresponds to the number of collecting containers and each of the second valves is connected to the inlet of one collecting container. According to another aspect, the at least one second valve is preferably a multi-way valve, wherein the number of ways of the multi-way valve preferably corresponds to the number of collecting containers.

The apparatus according to the invention further comprises a mounting device for mounting the sample, the mounting device comprising an inlet being connected to the first valve, an outlet, wherein the outlet of the mounting device is connected to the second valve, a sealable housing and a fixation apparatus for fixing the sample.

As used herein, the word "comprising" does not exclude other elements or steps, and the indefinite article "a" or "an" does not exclude a plurality. A single step may fulfil the functions of several features recited within the present invention.

Such an apparatus enables sterility and reproducibility within the exploitation of cells from a sample.

Preferably, the apparatus according to the invention further comprises a first fluid guiding device with inlets and an outlet, e.g. formed like a manifold, wherein each inlet of the first fluid guiding device is connected to one of the first valves, wherein the inlet of the mounting device is connected to the outlet of the first fluid guiding device.

Preferably, the apparatus according to the invention further comprises a second fluid guiding device, e.g. formed like a manifold, with an inlet and outlets, wherein each of the outlets of the second fluid guiding device is connected to the inlet of one collecting container, wherein the inlet of the second fluid guiding device is connected to the outlet of the mounting device.

Such fluid guiding devices within the apparatus according to the invention and as described herein provide a small loss of exploited cells, reproducible results and contribute to the sterility of the sample within the apparatus according to the invention.

Preferably, the mounting device according to the apparatus of the invention comprises a longitudinal extension being adaptable to the size of the sample, which enables that different sizes of samples can be used for the exploitation of cells. In particular, the mounting device according to the present invention is preferably adapted for mounting a tubular structure of a sample to the inlet and to the outlet in such a way that a fluid can flow from the inlet of the mounting device through the tubular structure to the outlet of the mounting device. The inlet and/or the outlet of the mounting device are closable, e.g., manually by a cap or a luer lock plug or remote-controlled by an actuator or a valve. The mounting device comprises a casing or housing which is a hermetically sealed housing such that the tubular sample is enclosed within the housing. This provides the further advantage that the tubular sample may be stored and/or treated within a well defined atmosphere within the housing. The housing of the mounting device comprises two openings, wherein the first opening may be used as an inlet for introducing a fluid (gas or liquid) to form a desired atmosphere within the housing. The two openings provide the further advantage that the first opening may be used as an inlet and the second opening may be used as an outlet. Accordingly, the atmosphere can be maintained or changed by a fluid flow from the inlet to the outlet during cell exploitation. The housing is preferably made from a cell and biologically compatible material that may preferably be easily sterilized. The outer diameter of the housing is preferably between 10 - 150 mm depending on the size of the tubular sample. The mounting device and/or the housing comprise preferably a length between 50-200mm.

The mounting device according to the invention further comprises a fixation apparatus being allocated to the inlet of the mounting device and to the outlet of the mounting device or both for removable fixing of the end(s) of the sample, which enables an easy handling of the sample within the apparatus of the invention. In particular, the fixation apparatus at the inlet and/or at the outlet may be formed as a connector or nozzle such that a biological sample or a biological sample with a tubular structure can be easily imposed on said connector or nozzle at the inlet and outlet side. Depending on the diameter of the biological sample or the biological sample with a tubular structure there may be provided different appropriate fixation apparatuses which may be mounted to the housing of the mounting device. According to a further aspect, the connector or nozzle may be formed in a tapered manner such that the connector may be used for a variety of samples or samples with tubular structures with different diameters.

The connectors are preferably made from a cell and biologically compatible material that may preferably be easily sterilized. The outer diameter of the connectors/nozzles are preferably between 0,1 - 100 mm depending on the size of the tubular sample.

According to a further aspect, the mounting device may comprise a plurality of fixation apparatuses at the inlet and/or at the outlet side, e.g. there may be provided two connectors at the inlet and at the outlet side of the mounting device which allows a parallel mounting of two tubular members. Moreover, for the exploitation of cells from an umbilical cord (also called the birth cord or funiculus umbilicalis) which comprises for humans normally two arteries (the umbilical arteries) and one vein (the umbilical vein), there may be provided three connectors at the inlet and at the outlet side.

The mounting device may be provided as a reusable device or as a disposable device. According to a further aspect, some parts of the mounting device are reusable and other parts may be provided as disposable parts. For instance the housing may be formed as a tube which allows easy cleaning and sterilization such that the housing may be reused.

Preferably, the apparatus according to the invention further comprises a heating device, a humidity device and/or a gas device for enabling temperature, humidity and/or gas transfer into the housing. These devices can be used for adjusting optimal conditions (preferably 37°C, Humidity > 95%, CO₂ level of 5%) for the viability of the sample and, thus, for the exploited cells.

In particular, the heating device is preferably located below or around the housing of the mounting device for solely heating the housing and the inner of the housing together with the sample. According to another aspect, however, the heating device may additionally or solely heat one or a plurality of the dispensing containers. According to another aspect, the heating device may additionally or solely heat one or a plurality of the collecting containers. According to still another aspect, the heating device may alternatively or additionally heat the fluid which can be introduced into the housing. The heating devices of the aforementioned aspects may be combined with the humidity device for enabling and controlling the temperature and humidity within the housing of the mounting device. Moreover, a gasing device may be coupled with one of the aforementioned devices to control the amount of a specific gas within the housing.

An advantage of the present invention is the automation of the sequence of different washing, rinsing and incubation steps of the cell exploitation. The apparatus of the present invention comprises a control unit for controlling the sequence of different method steps. In particular, the control unit may control the sequence on the basis of predetermined method steps (closed loop control unit). According to another preferred embodiment, during conducting the method the control unit receives information from outside, e.g., from the apparatus such that the control unit regulates and controls the sequence on the basis of this additional information.

Thus, it is preferred that the apparatus according to the invention further comprises a sensor for measuring an operating parameter, wherein the measured operating parameter is transferred to the control unit for controlling and regulating the operating parameter and/or the execution of the individual cell exploiting steps. Particularly, at least one operating parameter is selected from a group consisting of temperature, flow parameter, pressure, gassing, humidity. The at least one sensor can be used to measure operating parameters and, thus, may contribute to a cell exploitation being controllable within the apparatus of the invention.

The dispensing containers are preferably filled with fluid media which are selected from a group consisting of wash medium, stop medium, enzymatic medium and incubating medium. Thus, according to the present invention the appropriate fluid medium can be selected depending on the sample from which the cells are to be exploited and/or depending on the cell type(s) which is (are) to be exploited from the sample. Particularly, the fluid medium can be also a peptide medium comprising peptides or fragments thereof or, preferably a solution comprising target-specific catcher particles, more preferably beads containing a coating with target-specific molecules, most preferred antibodies. Target-specific, molecules as defined herein comprise inter alia cell-specific markers directed to the specific type of cell to be exploited by the methods of the present invention. By way of example, antibodies coated on the described particles / beads directed against CD73, CD90 and/or CD105 can be applied for the isolation of perivascular stem cells. The targeted cells bound to the surface of these catcher molecules can then separated from unbound cells through techniques known in the art, e.g. by centrifugation. Target cells can then be released via e.g. buffer treatment. In a further preferred aspect, the fluid medium can be also a peptide medium comprising a cell-specific amino acid sequence, which is able to bind to the surface of cells, for example to cell adhesion receptors.

According to the present invention, the fluid medium contacts the sample, wherein the fluid medium is stationary or dynamic in or around the sample. In particular, it is preferred that the fluid medium enters through the inlet of the mounting device into a tubular sample. The fluid medium may flow through the sample with a flow velocity > 0 (dynamic) or the flow velocity may be substantially zero, i.e., the medium is stationary within the tubular sample. Alternatively or additionally the fluid medium will flow or remain stationary outside-or around the sample, e.g., the sample and fluid medium are located within the closed housing.

A method for automated cell exploitation from a sample is envisaged within the present invention comprising the steps of providing a sample, mounting the sample into the mounting device of the apparatus according to the present invention and isolating cells from the sample by performing at least one washing step with fluid media using the media delivery arrangement wherein the control mit controls automatically at least the second valve, depending on a sensor signal. In the following the term "washing" will be used for any kind of rinsing or flushing of a fluid medium through and/or around the sample. In other words, the terms washing, rinsing or flushing are not limited to a specific fluid medium.

Preferably, a cell isolating step comprises a plurality of steps transferring the fluid media from the dispensing containers through the sample, wherein the setting of the valves, inlets and outlets is selected in a way that a media fluid is not mixed with another media fluid. In other words, the location of the valve or valves and the controlling of the valves is designed such that an undesired mixing of fluid media can be substantially avoided.

Particularly, the isolating step of the method according to the present invention comprises the following steps, preferably in the following sequence:
(a) A wash medium is transferred from one of the dispensing containers through the sample mounted in the mounting device to one of the collecting containers for cleaning the sample from waste, for example blood components such as erythrocytes, leucocytes and thrombocytes, or combination thereof.
(b) A first enzymatic medium or peptide medium, or a medium comprising specific beads containing a coating with target-specific molecules / antibodies as defined above is transferred from one of the dispensing containers into the sample mounted in the mounting device. Then, the first enzymatic medium or peptide medium is incubated within the sample for cell exploitation of cell type A for a certain period of time. The valves and/or the outlet of the mounting device are preferably closed during the incubation, i.e., a medium flow within the sample is substantially prevented.
(c) A stop medium is transferred from one of the dispensing containers into the sample mounted in the mounting device. Accordingly, the enzymatic reaction is substantially stopped. Furthermore, the exploited cells of cell type A are transferred into one of the collecting containers, wherein the outlet of the mounting device and/or the corresponding valve behind the mounting device is opened. The outlet of each of the dispensing containers containing the enzymatic or peptide medium and the wash medium, respectively, and the inlet of the collecting container containing the waste are closed.
(d) A second enzymatic medium or peptide medium is transferred from one of the dispensing containers into the sample mounted in the mounting device. Then, the second enzymatic medium or peptide medium is incubated within the sample for cell exploitation of cell type B for a certain period of time. The valves and/or the outlet of the mounting device are preferably closed during the incubation, i.e., a medium flow within the sample is substantially prevented.
(e) A stop medium is transferred from one of the dispensing containers into the sample mounted in the mounting device. Accordingly, the enzymatic reaction is substantially stopped. Furthermore, the exploited cells of cell type B are transferred into one of the collecting containers, wherein the outlet of the mounting device and/or the corresponding valve behind the mounting device is opened. The outlet of each of the dispensing containers containing the enzymatic media or peptide media and the wash medium, respectively, and the inlet of each of the dispensing containers containing the waste and cell type A are closed, respectively.

The steps of (a) to (e) may be repeated several times. Preferably the steps are repeated in a sequence from (a) to (e).

Particularly, cell type A or cell type B can be any type of cell existing in a biological sample.

Particularly, a biological sample can be derived from any type of tissue existing in a biological system.

Particularly, these cells can be eukaryotic cells. In case of a blood vessel as a sample, for instance, the isolated cells can be vascular cells like endothelial cells (e.g. cell type A) or fibroblasts or smooth muscle cells (e.g. cell type B). Particularly, in case of a umbilical cord (comprising in humans the Wharton-Sulze and normally two arteries such as the umbilical arteries and one vein such as the umbilical vein) as a sample, different types of cells can be isolated from the sample, wherein the isolated cells can be vascular cells like endothelial cells (e.g. cell type A), fibroblasts (e.g. cell type B) and/or perivascular stem cells (e.g. cell type C) from the Wharton-Sulze.
Preferably, the mounting device of the apparatus according to the present invention comprises at least three connectors at the inlet side, at the outlet side and at the fixation apparatus. Said mounting device can also be used within the method according to the invention. The sample can comprise at least three vessels and can be mounted to the at least three connectors of the fixation apparatus of the mounting device of the apparatus according to the present invention. This provides the advantage that a sample comprising at least three vessels like an umbilical cord can be mounted easily and, thus, enabling-sterility of the sample.

Preferably, the method according to the present invention further comprises the steps of filing the sample with a staining solution, removing the at least three vessels from the sample and isolating stem cells from the sample. According to the present invention, the staining solution can be a stained wax, silicone or acrylic solutions used for better visualization of the blood vessels. This visualization enables an easy removal of the vessels from the sample. The removal of the vessels from the sample according to the present invention can be carried out by a manual isolation or by an enzymatic destroy of the blood vessel. The isolation of the stem cells from the sample, preferably from the remaining part of the sample, like the Wharton-Sulze of the umbilical cord can be done by treating the Wharton-Sulze, free of blood vessels, with enzymatic or peptide medium as defined herein. Further, a method to isolate mesenchymal-like cells from Wharton's jelly of umbilical cord is described in Seshareddy et al., 2008, Methods Cell Biol. (86), 101-119.

Stem cell-specific markers for determining, e.g. the purity of the stem cells or the lineage / population (e.g. hematopoietic stem cells, neural, mesenchymal, stromal stem cells) of the stem cells obtained or obtainable by applying the method of the present invention can be effected by using immunohistochemical staining or FACS analysis. Appropriate markers for these stem cells are known in the art and encompass, e.g. antibodies directed against CD133, ABCG2 (ATP-binding cassette superfamily G member 2), Sca-1 (stem cell antigen 1), STRO-1, nestin, and p75 neurotrophin R. For the preferred perivascular stem cells, obtainable by performing the method of the present invention, antibodies against markers CD34, CD45 and/or HLA-DR should give a negative result while antibodies against CD73, CD90 and/or CD105 should give a positive signal.

The period of time for incubating the enzymatic medium, e.g. Trypsin-EDTA solution, collagenase type 1,2,3,4, hyalurodinase, elastase, papain, DNase, protease or combinations thereof or peptide medium within the sample, as used herein, is dependent on the cell type to be exploited from the sample as well as on the cell source. As a non-limiting example, in view of a blood vessel, a vein piece or a fragment thereof, the exploited cell type A can be endothelial cells and exploited cell type B can be fibroblasts. For the exploitation of endothelial cells, for instance, the period of time of incubating the enzymatic medium, e.g. Trypsin-EDTA solution, collagenase type 1,2,3,4, hyalurodinase, elastase, papain, DNase, protease or combinations thereof within the sample within the apparatus according to the invention is within a range of 1 to 40 minutes, preferably 1, 2, 3, 4, 5, 6, 7, 8, 9,10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 minute(s). For the exploitation of fibroblasts, for instance, the period of time of incubating the enzymatic medium, e.g. collagenase, within the sample within the apparatus according to the invention is within a range of 1 to 40 minutes, preferably 1, 2, 3, 4, 5, 6, 7, 8, 9,10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 minute(s).

Furthermore, the method of the present invention is preferably carried out, if not indicated otherwise, within a range of 18°C to 38°C. Most preferred is a temperature of 35°C, 36°C or 37°C, in particular for the washing and isolation steps disclosed herein.

In a preferred embodiment of the present invention the following method is applied to yield endothelial cells, and fibroblasts, preferably from human origin. A sample as defined herein is fixed via the mounting device (5) of the apparatus (1) of the present invention. After a washing step to remove unwanted blood components or contaminants, the sample is incubated usually for 10 to 30 min with an enzymatic medium comprising a 0,5 to 2% solution selected from trypsin-EDTA, collagenase, collagenase type 1,2,3,4, hyalurodinase, elastase, papain, DNase, protease or combinations thereof. Preferably, 1% of a collagenase or a 0.5% trypsin - EDTA (0.5 mM) solution at 37°C and about 5% CO₂ is used, with an incubation time about 20 min to about 25 min. In the next step, the enzymatic reaction is stopped with a stop medium as defined herein, e.g. M199 + FCS, and the ECs are collected in the collecting container (4) and can be further cultivated. In a subsequent step, the sample is incubated with a solution of 1 to 2%, trypsin, hyalurodinase, elastase, papain, DNase, protease or combinations thereof for about 5 to 30 min. Preferably, a solution of 2% collagenase, or collagenase type 1,2,3,4 is applied for about 10 min at about 37°C and about 5% CO₂. In the next step, the detachment is stopped with a stop medium used in accordance with the present invention, e.g. M199 + FCS and the FBs are collected in a further collecting container (4) and can be further cultivated.

In a further preferred embodiment of the present invention, the following method is applied to yield as a third cell population stem cells, preferably human stem cells. The Wharton-Sulze of the umbilical cord is used as a sample and fixed via the mounting device (5) of the apparatus (1) of the present invention. Blood vessels can be removed manually or enzymatically by, e.g. a treatment with the enzymatic medium or peptide medium as described herein. The Wharton-Sulze is incubated with an enzymatic medium selected from the group consisting of trypsin, collagenase type 1,2,3,4, hyalurodinase, elastase, papain, DNase, protease or combinations thereof. Preferably, the method is carried out for about 60 min with a solution comprising collagenase, most preferred collagenase Type I, for example at a concentration of 300 units/ml and hyaluronidase, e.g. obtained from ovine testes at a concentration of 1 mg/ml in a phosphate buffered saline (PBS), which may be supplemented with 3 mM CaCl₂. After a washing step, the sample is incubated with trypsin, collagenase type 1,2,3,4, hyalurodinase, elastase, papain, DNase, protease or combinations thereof. Preferably, the method is carried out for about 30 min with a solution containing Trypsin-EDTA (0.1%). In the next step the detachment is stopped with a stop medium used within a method in accordance with the present invention, e.g. M199 + FCS and the human stem cells are collected in a collecting container (4). Afterwards, the isolated stem cells can be further cultivated or differentiated.

In a further preferred embodiment, the following method is applied in order to yield endothelial cells, fibroblasts and stem cells. For this method, preferably an umbilical cord, most preferably a human umbilical cord is used as a sample. A part of the vena-umbilicalis is fixed via the mounting device (5) of the apparatus (1) of the present invention. ECs and FBs can be obtained by performing the method of the present invention as described herein, e.g. in the section for the exploitation of endothelial cells and fibroblasts, above. In a subsequent step, the vein and further blood vessels are enzymatically degraded by a incubation for 30 to 70 min with an enzymatic medium as described herein. Preferably, a solution of hyalurodinase and collagenase is used for 60 min. Preferably, the method is carried out at 37°C and about 5% CO₂. Alternatively, the vein and further blood vessels can be removed manually-from the sample, wherein the filling of the- blood vessels with a staining solution of the present invention is preferred in this alternative aspect of the present invention. After removal.of blood vessels, the sample may then be re-fixed, if necessary, in the apparatus of the present invention and the isolation of the stems cells can be effected as described herein, e.g. in the last preceding paragraph, preferably with an incubation step for about 60 min with collagenase Type I and hyaluronidase, followed by washing step and an elution step with a solution containing Trypsin-EDTA (0.1%) for about 30 min at 37°C or a medium comprising specific beads containing a coating with target-specific molecules for stem cells.

In an even more preferred aspect of the present invention, the sample comprises three or at least three blood vessels, whereby a umbilical cord and even more preferred a human umbilical cord is used. The vessels are mounted to the at least three connectors of the fixation apparatus (571, 572, 573) of the mounting device (5) of the apparatus (1) of the present invention. After the isolation of the vascular cells, i.e. endothelial cells and fibroblasts by applying the method steps of the present invention, the method further comprises the additional step of injecting a staining solution as defined herein, to aid in the removal of the at least three blood vessels from the sample. Alternatively, the at least three blood vessels are enzymatically degraded by the treatment with an enzymatic medium or peptide medium as defined and described herein. Thus, the method comprises a removal step of the at least three blood vessels. The method further comprises the subsequent step of isolating the stem cells from the remaining part of the sample including the Wharton-Sulze by applying the isolation steps as described herein for the isolation of stem cells.

The degree of purity of the appropriated cell type(s) exploited by the method according to the present invention can be analysed by using immunohistochemical staining or FACS analysis. The term "purity" defines the amount of an desired, exploited cell type (such as ECs) in relation to the total amount of exploited cells including an amount of contamination with, for example, undesired eukaryotic or prokaryotic cells. For example, as shown in Example 2 of the present invention, the EC cultures revealed a purity of >90% of ECs without any FB contamination. For example, as shown in Example 3 of the present invention, the FB cultures revealed a purity-of >95% of FBs without any smooth muscle cell (SMCs) contamination. Thus, the preferred "purity" or "degree of purity" by using the method according to the present invention may be in a range of 80% to 100%, preferably 85% to 100%, preferably 90% to 100%, " preferably 95% to 100%, preferably 97 % to 100%. Accordingly, the preferred "purity" or "degree of purity" by using the method according to the present invention may be 80%, 81%, 82%, 83%, 84%, 85%, 86%, 87%, 88%, 89%, 90%, 91%, 92%, 93%, 94%, 95%, 96%, 97, 98%, 99% or 100%.

The control unit controls automatically at least the second valve and preferably one device selected from the group consisting of the media delivery arrangement, the first valve, the second valve, the heating device or all thereof depending on the sensor signal. Further, the control unit can comprise a processing unit such as a computing system, e.g. a processor-based desktop computer or a processor based computing apparatus integrated into another device (embedded system). The computing system comprises computing means (processor) and memory means which are arranged to perform at least part of the steps of the method according to the invention. In particular, the computing system comprises means for automated exploitation of cells from a sample. With such a computing system, the automated exploitation of cells can be efficiently performed and reproduced with precision and small expenditure of time. Furthermore, the cell exploitation is well documented.

Particularly, the computing means and the memory means of the computing system are arranged to perform further steps of the method according to the invention as described above. Also, the computing system can comprise interface means for interacting with other components as used within the present invention.

Still another aspect of the invention relates to a computer program which is arranged to perform at least part of the steps of the method according to the invention when it is run on a computing system as mentioned above.

Preferably, the sensor within the method according to the invention induces an alert in case of a deviation of an operating parameter within the method. Such a sensor provides a controllability of the method according to the invention and, thus, contributing to conditions being optimal for the exploitation of cells, e.g. viability of exploited cells.

### DEFINITIONS

The technical terms and expressions used within the scope of this application are generally to be given the meaning commonly applied to them in the pertinent art of bioengineering, particularly in tissue engineering. All of the following term definitions apply to the complete content of- this application. The word "comprising" does not exclude other elements or steps, and the indefinite article "a" or "an" does not exclude a plurality. A single step may fulfil the functions of several features recited in the claims. The terms "essentially", "about", "approximately" and the like in connection with an attribute or a value particularly also define exactly the attribute or exactly the. value, respectively.

The term "exploitation of cells" or "exploiting cells" as used herein also relates to cell isolation, cell harvesting or enrichment of cells in a fluid media.

The term "sample" as used herein refers to a "biological sample", meaning a sample obtained from a biological subject. For example, this biological sample can be a tissue or organ sample, e.g. heart, liver, or preferably any biological sample with a tubular structure, like blood vessels, e.g. *Vena Saphena Magna,* vein and/or vein fragments, umbilical cord or fragments thereof, artery or lymphatics and fragments thereof.

In this context, cells obtained from a blood vessel by the use of the apparatus according to the invention can be any type of cell existing in such a sample as defined within the application. Preferably, these cells can be vascular cells, e.g. SMC, fibroblasts or endothelial cells. Cells obtained from the umbilical cord can be stem cells, in particular stem cells from the Wharton-Sulze. Fibroblasts and endothelial cells can also be obtained from the blood vessels of the umbilical cord.

The term "fluid medium", "fluid media", "fluid", "medium" or "media" as used within the present invention relates to cell suspension, enzymatic medium, wash medium, stop medium, incubating medium or peptide medium.

The term "medium" or "media" according to the present invention encompasses any suitable aqueous solution, e.g. distilled water, preferably a buffered saline solution like PBS, HEPES, TBS, etc., or cell culture media commonly used in cell biology, e.g. Minimum Essential Medium (MEM), RPMI, medium 199, etc. The term "enzymatic medium" defines any enzyme to be used for the detachment of cells from a substrate. The choice of the enzymatic medium depends on the cell type to be exploited from the sample. For example, without a limitation, the enzyme to be used for the exploitation of fibroblasts or endothelial cells from a blood vessel is selected from a group consisting of Trypsin, Trypsin/ ethylenediaminetetraacetic acid (EDTA), collagenase, collagenase type 1,2,3,4, hyalurodinase, elastase, papain, DNase, protease or combinations thereof in an aqueous solution or medium as defined above. Preferably, HSA solution (Human serum albumin) or PBS are used as an aqueous solution. For the isolation of stem cells, trypsin, collagenase, collagenase type 1,2,3,4, hyalurodinase, elastase, papain, DNase, protease or combinations thereof can inter alia be used.
The term "wash medium" relates to a compound or composition or medium as defined herein for the removal of waste from the sample, like blood-or blood components. In particular, such a composition can consist of heparin and cell medium (M199).

The term "stop medium" as used herein relates to a composition stopping the enzymatic reaction. In particular, such a composition can be cell- medium (M199) with fetal calf serum (FCS) or human serum.

The term "incubating medium" relates to a medium as defined herein, e.g. cell medium (M199) or PBS which preserves the samples for the period of isolation. Further, the incubating medium can be also used for wetting of the sample in order to provide the vitality of the cells within the sample.

The term "peptide medium" relates to a fluid medium, fluid media, fluid, medium or media and comprises peptides or a fragment thereof. Preferably, the peptide medium comprises specific beads or specific catcher particles containing a coating with target-specific molecules / antibodies. This peptide or fragment thereof can be natural or synthetical and comprises an amino acid sequence, preferably a cell-specific amino acid sequence, which is able to bind to the surface of cells, preferably to cell adhesion receptors. Based on this binding, the cells within the sample according to the invention are able to detach from a substrate, wherein the substrate can be either biological, like another cell layer, or synthetical. Thus, such a peptide or fragment thereof can be used solely or in combination with an enzyme in order to detach and exploit cells from the sample.

The term "control unit" as used herein also relates to an open-loop control unit, a closed-loop control unit or processing unit or a combination thereof. Further, one or more components of the control unit can be a module and are connected to each other.

The term "dispensing container" as used herein also refers to a module containing fluid medium either being disposable or recyclable.

The term "fluid guiding device" relates to a tubing connection, which connects one or more components within the apparatus according to the invention to each other.

The term "collecting container" as used herein also refers to a module either being disposable or recyclable. This container, as defined herein above, can be used for separate storage of fluid medium.

The term "media delivery arrangement" as used herein refers to a pump device or a syringe pump device, wherein one or more syringes are connected to one or more pumps. Preferably the apparatus according to the invention comprises four or more syringes, each connected to one or more pumps.

The term "deviation" is used herein in the context of operating parameters and represents the difference between the measured value and the set-value.

The term "EC" or "ECs" as used herein is an abbreviation and stands for endothelial cells.

The term "FB" or "FBs" as used herein is an abbreviation and stands for fibroblasts.

The term "FCS" as used herein is an abbreviation and stands for fetal calf serum.

The term "IHC" as used herein is an abbreviation and stands for immunohistochemistry. As used herein, immunohistochemistry relates to a process of localizing antigens (e.g. proteins) in cells of a tissue section exploiting the principle of antibodies (monoclonal or polyclonal) binding specifically to antigens in biological tissues by the use of markers that are either fluorescent dyes or enzymes, especially peroxidase. The mode for carrying out this process is known to the skilled person.

The term "AEC substrate kit" as used herein is an abbreviation and stands for Amino-Ethylcarbazole kit

The term "SMC" or "SMCs" as used herein is an abbreviation and stands for smooth muscle cells.

The term "RT" as used herein is an abbreviation and stands for room temperature, preferably 20-22 °C.

### BRIEF DESCRIPTION OF THE DRAWINGS

The apparatus according to the present invention is described in more detail herein below by way of exemplary embodiments and with reference to the attached non-limiting drawings, in which:
- Fig. 1: shows a schematic diagram of an apparatus according to the present invention;
- Figs. 2a-d: show schematic views of an apparatus according to the present invention from different perspectives;
- Fig. 3: shows a schematic view of an embodiment of a mounting device according to the present invention;
- Fig.4: shows a schematic perspective view of a further embodiment of an apparatus according to the present invention;
- Fig. 5: shows a perspective partly explosion view of a mounting device with a housing according to the present invention;
- Fig. 6: shows a schematic view of a further embodiment of a mounting device according to the present invention;
- Fig. 7: shows a schematic exterior view of the further embodiment of a mounting device-according to the present invention;
- Fig. 8: shows the IHC analysis of exploited ECs;
- Fig. 9: shows the IHC analysis of exploited FBs; and
- Fig. 10: shows the result of the angiogenesis test for exploited ECs.
- Fig. 11: shows a schematic view of a further embodiment of a mounting device with a housing according to the present invention;
- Fig. 12: shows a schematic view of the further embodiment of a mounting device with a housing according to the present invention;
- Fig. 13: shows a schematic exterior view of the further embodiment of a mounting device with a housing according to the present invention;

### DETAILED DESCRIPTION OF THE PRESENT INVENTION AND PREFERRED EMBODIMENTS

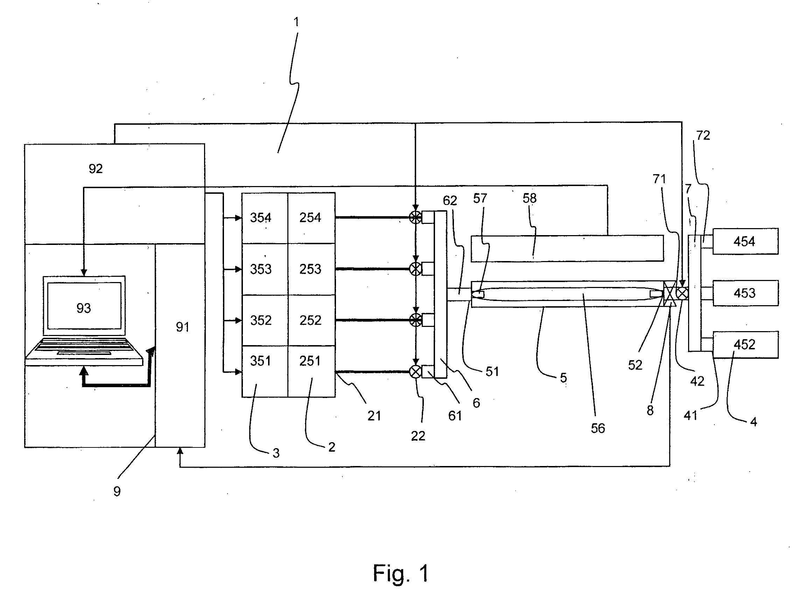

Fig. 1 shows a schematic diagram of an apparatus 1 for automated exploitation of cells from a sample. The apparatus comprises a plurality of dispensing containers 2, a media delivery arrangement 3. associated with the dispensing containers 2 and a plurality of collecting containers 4. For instance, this particular embodiment comprises four dispensing containers 251, 252, 253, 254 for the separated storage of fluid media. Each dispensing container comprises an outlet 21 wherein the media delivery 3 arrangement comprises the four media delivery arrangements 351, 352, 353, 354 associated with the four dispensing containers 251, 252, 253, 254, respectively. Four valves 22 are connected to the outlets 21 of the four dispensing containers 251, 252, 253, 254, respectively. As mentioned above, each of the plurality of outlets may be provided with an individual valve. According to the present invention the plurality of outlets of the containers may also be connected to a multi-way valve which also allows individual controlling of the flow from the individual outlets. The same is true for the inlets of the collecting containers.

For instance the apparatus 1 of Fig. 1 comprises three collecting containers 452, 453, 454 for the collection of fluid media, each collecting container 452, 453, 454 comprising an inlet 41, wherein a single multi-way second valve 42 is connected to the inlets 41 of the collecting containers via a manifold.

Fig. 1 shows between the dispensing and collecting containers a mounting device 5 for mounting the sample, the mounting device comprising an inlet 51 being connected to the four valves 22 being the first valve, and an outlet 52, wherein the outlet 52 of the mounting device is connected to the second valve 42. The apparatus 1 according to the invention, further comprises a first fluid guiding device 6 in form of a manifold with four inlets 61 and one outlet 62, wherein each inlet 61 of the first fluid guiding device 6 is connected to the each of the four valves 22 being the first valve 22, wherein the inlet 51 of the mounting device is connected to the outlet 62 of the first fluid guiding device.

The apparatus 1 according to the invention further comprises a second fluid guiding device 7 in form a manifold with an inlet 71 and three outlets 72, wherein each of the three outlets 72 of the second fluid guiding device 7 is connected to an inlet 41 of the three collecting containers 452, 453, 454, respectively. The inlet 71 of the second fluid guiding device 7 is connected to the outlet 52 of the mounting device.

The mounting device comprises a longitudinal extension 56 being adaptable to the size of the sample. Further, the mounting device comprises a fixation apparatus 57 being allocated to the inlet 51 of the mounting device or to the outlet 52 of the mounting device or both for removably fixing the end(s) of the sample.

The apparatus according to the invention preferably comprises a heating device 58 for enabling temperature transfer into the apparatus, particularly into the mounting device. Preferably, the heating device can actively heat and measure the transferred temperature in the apparatus, particularly in the mounting device, at the same time. Thus, a constant temperature condition is enabled within the present invention.

The apparatus may further comprise a sensor 8 for measuring at least one operating parameter, wherein the- measured operating parameter is transferred to. a control unit 9 for controlling and regulating the operating parameter. The control unit 9 may further comprise a processing unit 93, an open-loop unit (controller) 92 and a closed-loop unit (regulator) 91. Preferably, the open-loop unit 92 is connected to the first valve 22 and/or to the second valve 42 for controlling the valves. The closed-loop unit 91 is connected to the sensor 8 for regulating the apparatus on the basis of the measured parameter. This set-up enables an automated process within the present invention.

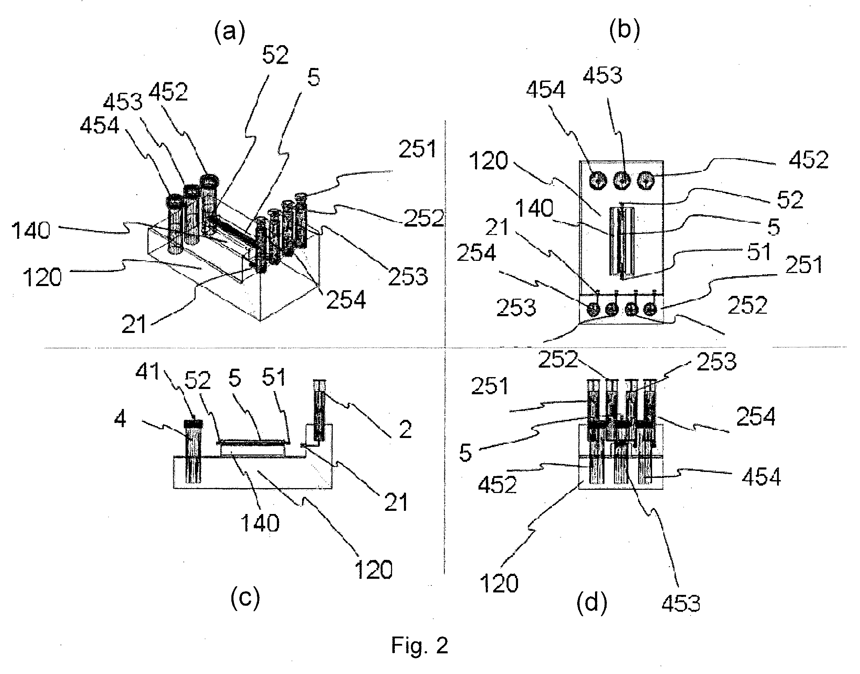

Figures 2a to 2d show schematic views of an embodiment of the apparatus 1 with the mounting device 5, the dispensing containers 2, the collecting containers 4 and further components according to the present invention from different perspectives. In particular, the four depicted dispensing containers 251, 252, 253, 254 with corresponding media delivery arrangements 351, 352, 353, 354 are provided in form of four syringes containing fluid media. It is preferred that each of the syringes is individually exchangeably mounted to the apparatus 1 which provides a flexibly adaptable apparatus 1.

The apparatus is preferably fixed to an apparatus carrier device 120. Furthermore, the mounting device 5 may be further fixed to the carrier device 120 via a support device 140. The supporting device may comprise a heating device 58 for heating the mounting device 5 to a desired temperature.

The four dispensing containers 251, 252, 253, 254 are preferably positioned with regard to the gravitational field above the other components in order to support a constant flow profile within the mounting device 5. The remaining components shown in Figure 2 are fully shown in Figure 1.

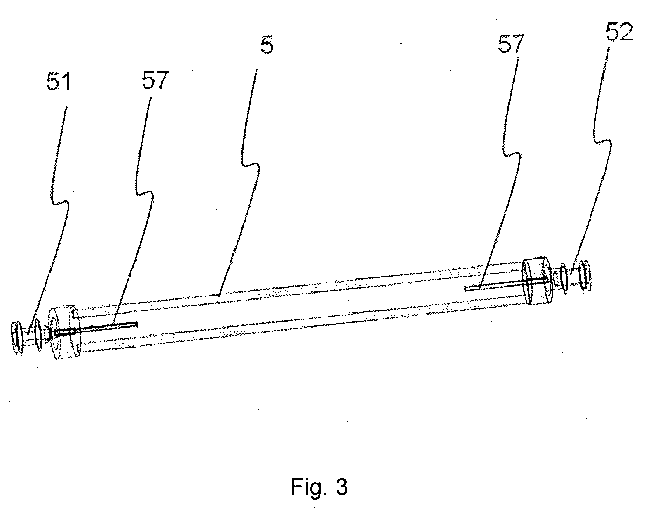

Figure 3 shows an enlarged view of a mounting device 5 according to the present invention. The mounting device 5 comprises at least an inlet 51 and an outlet 52 for connecting the mounting device 5 to the dispensing container and the collecting container, respectively. It is preferred to provide the inlet 51 and the outlet 52 each with a fixation apparatus 57 for removable fixing of the end(s) of the sample to the inlet 51 and the outlet 52 of the mounting device 5, respectively. The fixation apparatus 57 are formed as nozzles such that a tubular structure of the sample can be easily imposed on said nozzles 57. The inlet 51 is spaced from the outlet 52 by the two depicted rods. The rods are preferably fixed to maintain a fixed distance between the inlet 51 and the outlet 52. According to another aspect of the invention, the distance between the inlet 51 and the outlet 52 may be adjusted.

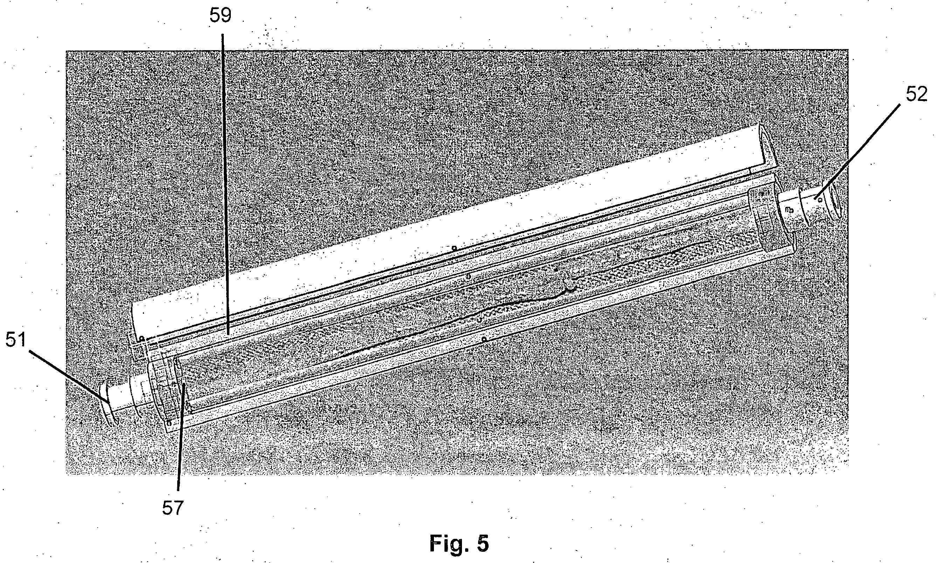

Figure 5 shows a mounting device 5 with a housing 59 around the tubular sample. For illustrative purposes, the housing 59 is shown in a partly exploded view, whereas the housing is preferably closable, more preferably hermetically sealable such that the sample can be stored and/or treated within a desired atmosphere inside the housing.

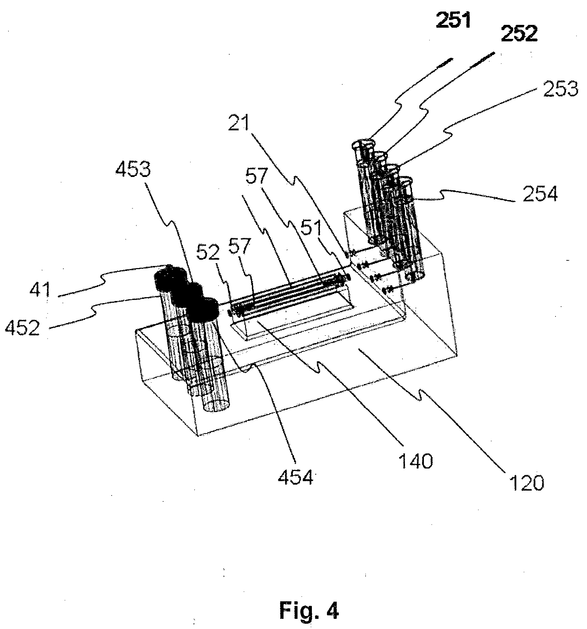

Figure 4 represents an angular view of Figure 2 including the fixation apparatus 57 being allocated to the inlet 51 and to the outlet 52 of the mounting device 5 as shown in Figure 3.

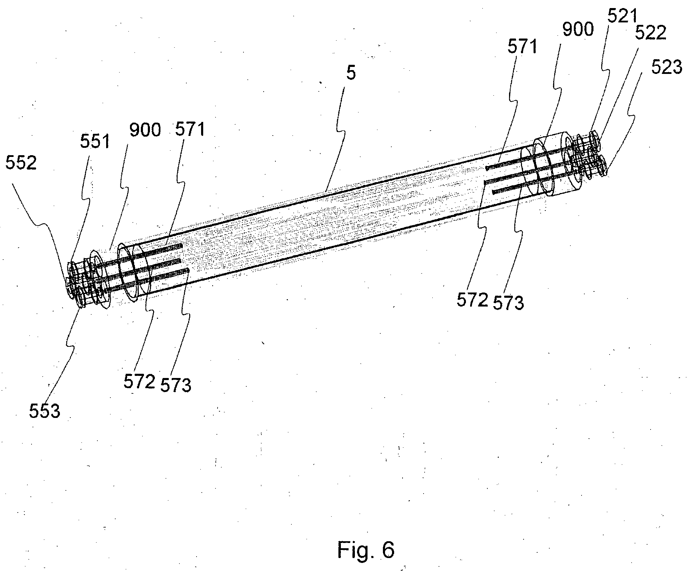

Figure 6 shows a schematic view of a further embodiment of a mounting device according to the present invention. The mounting device 5 comprises at least three inlets 551, 552, 553 and outlets 521, 522, 523 for connecting the mounting device 5 to the dispensing containers and the collecting containers, respectively. It is preferred to provide the at least 3 inlets 551, 552, 553 and outlets 521, 522, 523 each with fixation apparatuses 571, 572, 573 for removable fixing of the end(s) of the sample to the inlets 551, 552, 553 and outlets 521, 522, 523 of the mounting device 5, respectively. The fixation apparatuses 571, 572, 573 are formed as nozzles such that a tubular structure of the sample can be easily imposed on said nozzles 571, 572, 573. The inlets 551, 552, 553 are spaced from the outlets 521, 522, 523 by the two depicted rods 900. The rods 900 are preferably fixed to maintain a fixed distance between the inlets 551, 552, 553 and the outlets 521, 522, 523. According to another aspect of the invention, the distance between the inlets 551, 552, 553 and the outlets 521, 522, 523 may be adjusted.

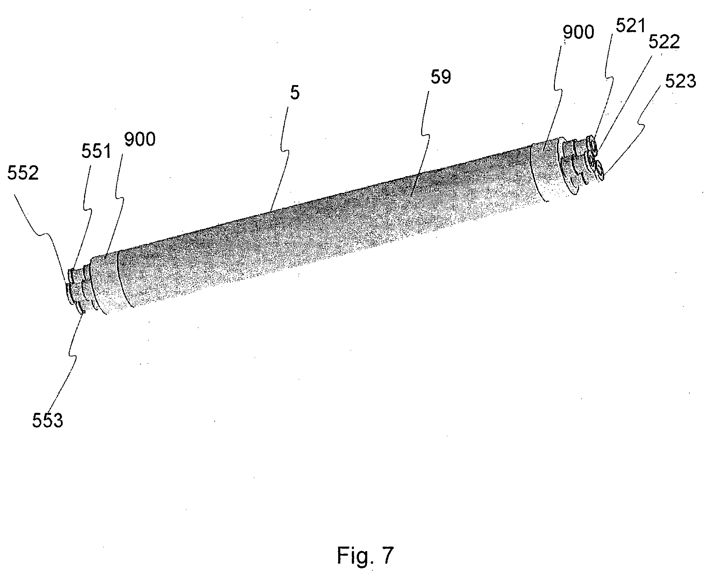

Figure 7 shows a schematic exterior view of a further embodiment of a mounting device -according to the present invention. The mounting device 5 comprises at least three inlets 551, 552, 553 and outlets 521, 522, 523 for connecting the mounting device 5 to the dispensing containers and the collecting containers, respectively. The inlets 551, 552, 553 are spaced from the outlets 521, 522, 523 by the two depicted rods 900. According to another aspect of the invention, the distance between the inlets 551, 552, 553 and the outlets 521, 522, 523 may be adjusted. Preferably, either the rods 900 or the housing 59 or both are fixed to maintain a fixed distance between the inlets 551, 552, 553 and the outlets 521, 522, 523.

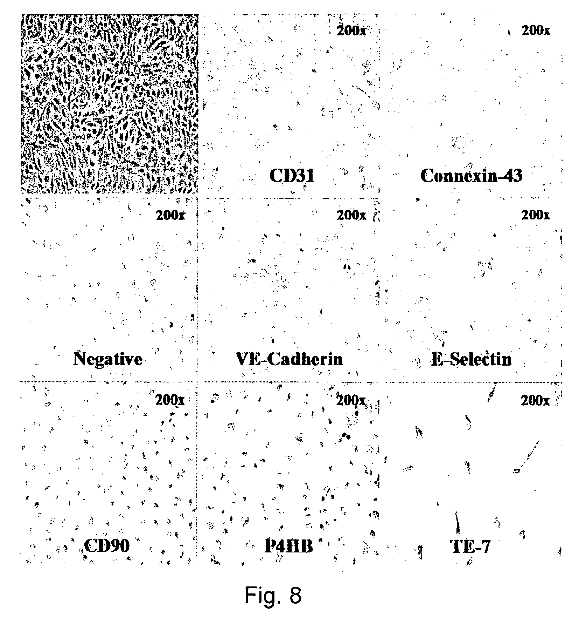

Figure 8 shows the immunohistochemical analysis of exploited ECs, the indicated specific marker molecules are explained below.

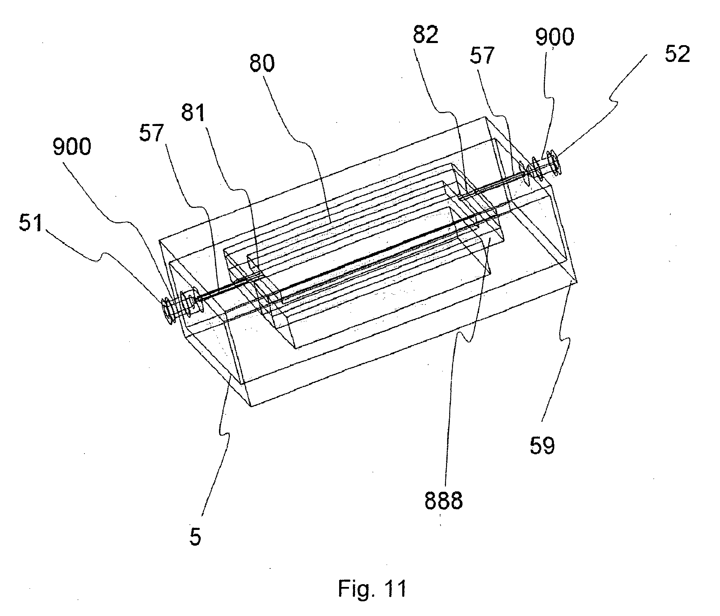

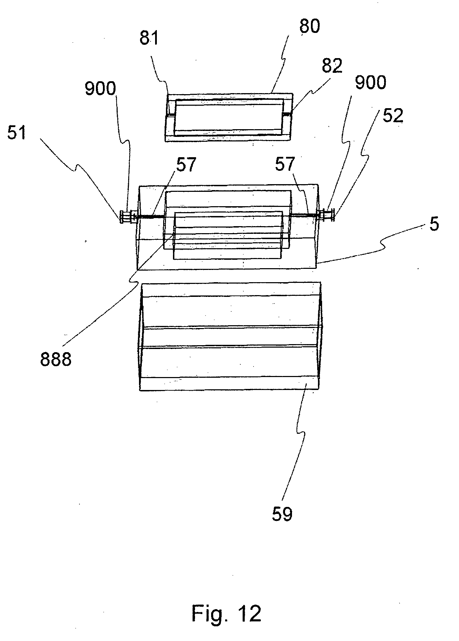

Figures 11 and 12 show a schematic view of a further embodiment of a mounting device with a housing according to the present invention. The mounting device 5 comprises at least one inlet 51 and outlet 52 for connecting the mounting device 5 to the dispensing containers and the collecting containers, respectively. It is preferred to provide the at least one inlet 51 and outlet 52 each with fixation apparatus 57 for removable fixing of the end(s) of the sample to the inlet 51 and outlets 52 of the mounting device 5, respectively. Preferably, the fixation apparatus 57 is formed as nozzle such that a tubular structure of the sample can be easily imposed on said nozzle. The mounting device 5 further comprises a shoulder 888 for inserting a cartridge 80. The shoulder 888 is formed such as the cartridge 80 to be inserted can be positioned planar and flushed with the inlet 51 and outlet 52 of the mounting device 5. The cartridge 80 comprises two notches 81, 82, each for connecting the inlet 51 and outlet 52 of the fixation apparatus 57. Preferably, the cartridge 80 is formed as a frame with an opening for enabling the fixation of large-sized samples within the cartridge 80. The inlet 51 is spaced from the outlet 52 by the two depicted rods 900. The rods 900 are preferably fixed to maintain a fixed distance between the inlet 51 and the outlet 52. According to another aspect of the invention, the distance between the inlet 51 and the outlet 52 may be adjusted. Preferably, the mounting device 5 is surrounded by a housing 59, whereas the housing 59 is preferably closable, more preferably hermetically sealable such that the sample can be stored and/or treated within a desired atmosphere inside the housing.

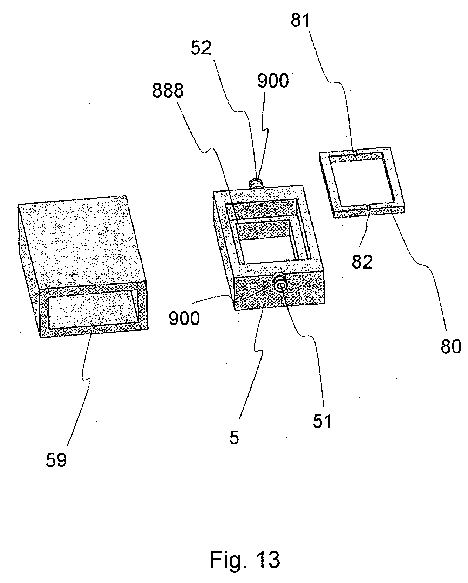

Figure 13 shows a schematic exterior view of the further embodiment of a mounting device with a housing according to the present invention. The mounting device 5 comprises at least one inlet 51 and outlet 52 for connecting the mounting device 5 to the dispensing containers and the collecting containers, respectively. The mounting device 5 further comprises a shoulder 888 for inserting a cartridge 80. The shoulder 888 is formed such as the cartridge 80 to be inserted can be positioned planar and flushed with the inlet 51 and outlet 52 of the mounting device 5. The cartridge 80 comprises two notches 81, 82, each for connecting the inlet 51 and outlet 52 of the fixation apparatus 57. Preferably, the cartridge 80 is formed as a frame with an opening for enabling the fixation of large-sized samples within the cartridge 80. The inlet 51 is spaced from the outlet 52 by the two depicted rods 900. The rods 900 are preferably fixed to maintain a fixed distance between the inlet 51 and the outlet 52. According to another aspect of the invention, the distance between the inlet 51 and the outlet 52 may be adjusted. Preferably, the mounting device 5 is surrounded by a housing 59, whereas the housing 59 is preferably closable, more preferably hermetically sealable such that the sample can be stored and/or treated within a desired atmosphere inside the housing.

The following Examples illustrate the invention:

### EXAMPLES

### Example 1: Cell isolation from Saphenous vein:

Saphenous vein pieces are cannulated from both sides and incubated with 1% collagenase or Trypsin - EDTA solution (10x) for 25 min at 37°C and 5% CO₂. Thereafter, the enzymatic reaction is stopped with a stop medium (M199 + FCS) and the cell suspension is centrifuged at 1000 RPM and 4°C for 10 min, resuspended in ECs growth medium, and plated in cell culture flasks. For FBs isolation, same vein pieces are filled with 2% collagenase for 30 min, stopped with a stop medium, centrifuged at 1000 RPM and 4°C for 10 min, resuspended in FBs growth medium, and plated in cell culture flasks.

### Example 2: Cell isolation from umbilical cord:

Vein pieces of umbilical cord are cannulated from both sides and incubated with 1% collagenase or Trypsin - EDTA solution (10x) for 15 min at 37°C and 5% CO₂. Thereafter, the enzymatic reaction is stopped with a stop medium (M199 + FCS) and the cell suspension is centrifuged at 1000 RPM and 4°C for 10 min, resuspended in ECs growth medium, and plated in cell culture flasks. For FBs isolation, same vein pieces are filled with 2% collagenase for 20-30 min, stopped with a stop medium, centrifuged at 1000 RPM and 4°C for 10 min, resuspended in FBs growth medium, and plated in cell culture flasks.

### Example 3: Immunohistochemistry

Cell specimens are transferred to culture slides (4- or 8-well), cultivated and finally fixed in acetone at -20°C. Endogenous peroxidase is blocked with a 10 min incubation of 3% hydrogen peroxide. Slides are then washed and incubated at RT for 30 min with monoclonal antibodies for CD31^{(a)}, CD144^{(b)}, P4hb^{(c)}, TE-7^{(d)}, Collagen IV^{(e)}, α-actin^{(f)}, Myosin^{(g)}, Desmin^{(h)}, CD62E⁽ⁱ⁾, MS-X Connexin 43^{(j)}, Elastin^{(k)}, CD90^{(l)} and polyclonal anti-FN^{(m)} (see Table 1). FBs with a, c, d, e, g, h, j, k, l, m; ECs with a, b, c, d, i, j, l.

The targets such as antigens bounded by the antibodies used according to the present invention serve as specific markers for the identification of different cell types within immunohistochemical analysis:
CD31 is expressed on all continuous endothelia such as those of veins, CD144 is a specific vascular EC-marker, P4hb is a typical fibroblast marker, CD90 is used as a specific marker to identify fibroblasts contaminations in cell culture and to recognize activated endothelial cells, Human Thymic FBs (TE-7) is a marker that specifically recognizes fibroblasts surrounding blood vessels, Type IV Collagen is synthesized by FBs, smooth muscle α-actin represents a useful tool in distinguishing SMCs from FBs, smooth muscle myosin serves as reliable marker to characterize SMCs, Desmin is used to distinguish SMCs from activated FBs, CD62E is a cell-surface protein highly expressed by activated ECs, Connexin 43 is exclusively expressed by ECs and SMCs, Elastin is specifically used as FBs-marker, FN is found in most tissues and body fluid and binds specifically to native collagen. For the identification of perivascular stem cells, antibodies against markers CD34, CD45 and/or HLA-DR should give a negative result while positive signal with antibodies against CD73, CD90 and/or CD105.

**Table 1: Antibodies used for cell culture slides.**

| Antibody | Dilution | Incubation |
|---|---|---|
| Monoclonal CD31 | 1:30 | 30 min at RT |
| Monoclonal CD144 | 1:12 | 30 min at RT |
| Monoclonal P4hb | 1:100 | 30 min at RT |
| Monoclonal anti-FB | 1:150 | 30 min at RT |
| Monoclonal Collagen IV | 1:600 | 30 min at RT |
| Monoclonal α-actin | 1:200 | 30 min at RT |
| Polyclonal FN | 1:600 | 30 min at RT |
| Monoclonal Myosin | 1:100 | 30 min at RT |
| Monoclonal Desmin | 1:200 | 30 min at RT |
| Monoclonal CD62E | 1:40 | 30 min at RT |
| Monoclonal MS X Connexin 43 | 1:350 | 30 min at RT |
| Monoclonal CD90 | 1:400 | 30 min at RT |
| Monoclonal Elastin | 1:5000 | 30 min at RT |

The visualising of an antibody-antigen interaction can be accomplished by using immunoperoxidase staining. For example, an antibody is conjugated to an enzyme, such as peroxidase, that can catalyse a colour-producing reaction. After developing the stain by adding the chemical substrate, the distribution of the stain can be examined by light microscopy.

Slides for P4hb and TE-7 are washed and primarily blocked for 30 min with 15% swine serum plus 5% human serum. The primary antibody is localized using the Dakocytomation peroxidase conjugated EnVision rb/m Dual link system. Specimens were then washed and incubated with AEC substrate kit for 10 min. Hemalaun is used as the nuclear counter-stain. As negative controls, cells are stained by replacing the primary antibodies with PBS. The intensity of IHC staining is then analyzed and classified as high (+++), medium (++), low (+) sand absent (0) (see. Table 2 and Table 3).

**Table 2: Classification of immunohistochemical staining of exploited ECs.**

| CD31 | CD144 | Connexin 43 | CD62E | CD90 | P4hb | TE-7 |
|---|---|---|---|---|---|---|
| +++ | +++ | + | 0/+ | 0 | 0 | 0 |

IHC staining of EC cultures revealed a purity of >90% of ECs without any FB contamination (see Figure 8). Low positive connexin-43 reaction showed the ability of the isolated ECs to communicate via the gap junctions.

**Table 3: Classification of immunohistochemical staining of exploited FBs.**

| P4hb | TE-7 | CD90 | Fibronectin | Elastin | Collagen IV | Desmin | α-actin | SMC-Myosin | CD31 |
|---|---|---|---|---|---|---|---|---|---|
| +++ | +++ | +++ | +++ | 0/+ | ++ | 0 | ++ | ++ 0/+ | 0 |

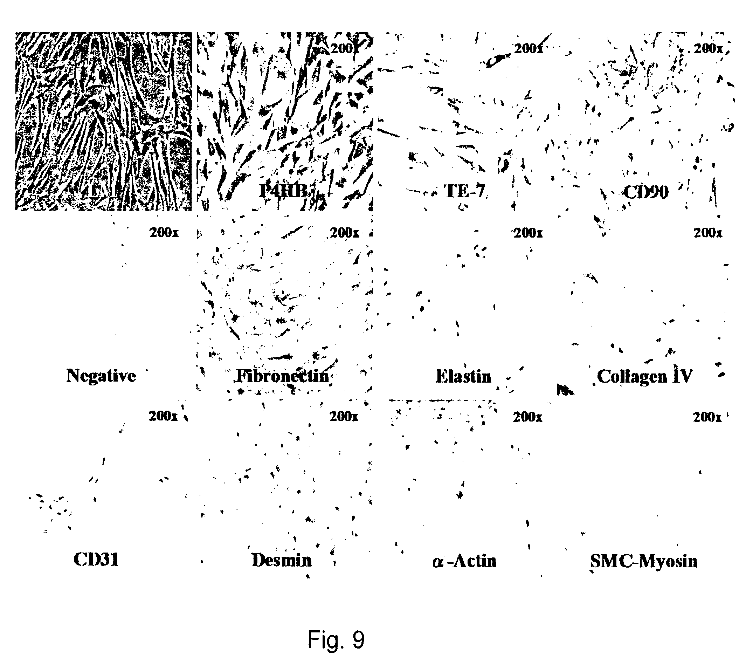

The IHC results of FB cultures showed a purity > 95% of FBs with little to no contamination with SMCs (see Figure 9).

### Example 4: Angiogenesis of exploited cells

One of the specific tests for angiogenesis is the measurement of the ability of ECs to form three-dimensional structures. However, cells of nonendothelial origin, such as FBs, may also exhibit a response to Matrigel (Auerbach et al., 2003). Therefore, for negative control, it is not feasible to measure the tube formation achieved by FBs.

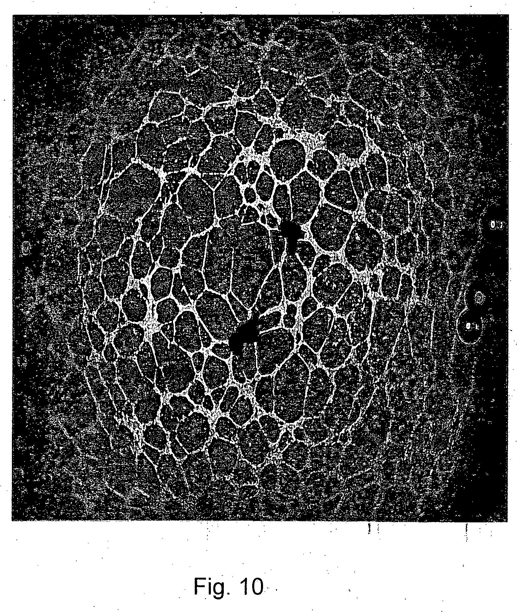

Matrigel is thawed on ice in the 4°C fridge and added (200 µl/well) to the wells by keeping culture plates on ice: Thereafter, Matrigel is allowed to gel at room temperature for 15 min. ECs (100 000 cells/well) are then placed in the well, along with cell medium. Cell movement are scored over 24h under physiological conditions 37°C and 5% CO₂ The results of the specific tests showed the ability of ECs to form tubules already after 4h (see Figure 10).

While the invention has been illustrated and described in detail in the drawings and foregoing description, such illustration and description are to be considered illustrative or exemplary and not restrictive.

The invention also covers all further features shown in the Figures individually although they may not have been described in the afore or following description. Furthermore, in the claims the word "comprising" does not exclude other elements or steps, and the indefinite article "a" or "an" does not exclude a plurality. A single step may fulfil the functions of several features recited in the claims. The terms "essentially", "about", "approximately" and the like in connection with an attribute or a value particularly also define exactly the attribute or exactly the value, respectively. A computer program may be stored/distributed on a suitable medium, such as an optical storage medium or a solid-state medium supplied together with or as part of other hardware, but may also be distributed in other forms, such as via the internet or other wired or wireless telecommunication systems. Any reference signs in the claims should not be construed as limiting the scope.

## Claims

1. Apparatus (1) for an automated exploitation of cells from a sample, comprising:
a plurality of dispensing containers (2) for the separated storage of fluid media, each dispensing container comprising an outlet (21) wherein a media delivery arrangement (3) is associated to each of the dispensing containers (2) and a first valve (22) is connected to each of the outlets (21),
a plurality of collecting containers (4) for the collection of fluid media, each collecting container (4) comprising an inlet (41), wherein a second valve (42) is connected to the inlet (41) of each collecting container,
a control unit (9) controlling at least the second valve (42) for the execution of individual cell exploiting steps, and
a mounting device (5) for mounting the sample, the mounting device comprising:
a housing (59) being sealable such that the sample is stored/treated within a defined atmosphere,
a closable inlet (51) being connected to the first valve (22), and a closable outlet (52), wherein the outlet (52) of the mounting device is connected to the second valve (42), and
a fixation apparatus (57) at the inlet (51) of the mounting device and the outlet (52) of the mounting device for removable fixing of the end(s) of the sample.

2. The apparatus according to claim 1, wherein the number of valves (22, 42) corresponds to the number of dispensing containers (2) and each of the first valves (22) is connected to the outlet (21) of one dispensing container and each dispensing container (2) is connected to one of the first valves (22).

3. The apparatus according to claim 2, further comprising a first fluid guiding device (6) with inlets (61) and an outlet (62), wherein each inlet (61) of the first fluid guiding device (6) is connected to one of the first valves (22), wherein the inlet (51) of the mounting device is connected to the outlet (62) of the first fluid guiding device.

4. The apparatus (1) according to any one of the preceding claims, further comprising a second fluid guiding device (7) with an inlet (71) and outlets (72), wherein each of the outlets (72) of the second fluid guiding device is connected to the inlet (41) of one collecting container, wherein the inlet (71) of the second fluid guiding device is connected to the outlet (52) of the mounting device.

5. The apparatus according to any one of the preceding claims, wherein the mounting device comprises a longitudinal extension (56) being adaptable to the size of the sample.

6. The apparatus according to any one of the preceding claims, the apparatus further comprising a heating device (58) for heating parts of the apparatus.

7. The apparatus according to any one of the preceding claims, wherein said apparatus further comprises a humidity device for enabling and/or controlling the humidity within the housing (59) and/or a gasing device for providing and/or controlling the gas composition within the housing (59).

8. The apparatus according to any one of the preceding claims, further comprising a sensor (8) for measuring an operating parameter, wherein the measured operating parameter is transferred to a control unit (9) for controlling and regulating the operating parameter, preferably wherein the at least one operating parameter is selected from a group consisting of temperature, flow parameter, pressure, gassing, humidity.

9. The apparatus according to any one of the preceding claims, wherein the fluid media are selected from a group consisting of wash medium, stop medium, enzymatic medium and incubating medium.

10. A method for automated cell exploitation from a sample comprising the steps of providing a sample,
mounting the sample into the mounting device (5) of the apparatus (1) of any one of the preceding claims and
isolating cells from the sample by performing a washing step with media using the media delivery arrangement (3)
wherein the control unit (9) controls automatically at least the second valve (42), depending on a sensor signal.

11. The method according to claim 10, wherein the isolating step comprises a plurality of steps transferring the fluid media from the dispensing containers through the sample, wherein the setting of the valves (22, 42), inlets (41, 51, 61, 71) and outlets (21, 52, 62, 72) is selected in a way that a media fluid is not mixed with another media fluid, and/or wherein the media comprises beads or catcher particles containing a coating with target-specific molecules.

12. The method according to claim 10 or 11, wherein a control unit (9) further controls automatically at least one device selected from the group consisting of media delivery arrangement (3), the first valve (22), heating device (58) or all thereof depending on the sensor signal, and/or wherein the sensor induces an alert in case of a deviation of an operating parameter within the method.

13. The method according to any one of claims 10 to 12, wherein the mounting device (5) of the apparatus (1) of any one of the preceding claims comprises at least three connectors at the inlet side (551, 552, 553), outlet side (521, 522, 523) and at the fixation apparatus (571, 572, 573),wherein the sample preferably comprises at least three vessels and is mounted to the at least three connectors of the fixation apparatus (571, 572, 573) of the mounting device (5) of the apparatus (1) of any one of the preceding claims.

14. The method according to any one of claims 10 to 13 further comprising the steps of filling the sample after vascular cell isolation with a staining solution, removing the at least three vessels from the sample and isolating stem cells from the sample.

## Patentansprüche

1. Vorrichtung (1) zur automatisierten Verwertung von Zellen einer Probe, enthaltend:
mehrere Abgabebehälter (2) für die getrennte Lagerung fluider Medien, wobei jeder Abgabebehälter einen Auslass (21) aufweist, wobei jedem der Abgabebehälter (2) eine Medienförderanordnung (3) zugeordnet ist und wobei ein erstes Ventil (22) mit jedem der Auslässe (21) verbunden ist,
mehrere Sammelbehälter (4) für die Sammlung fluider Medien, wobei jeder Sammelbehälter (4) einen Einlass (41) aufweist, wobei ein zweites Ventil (42) mit dem Einlass (41) jedes Sammelbehälters verbunden ist,
eine Steuereinheit (9), die zumindest das zweite Ventil (42) für die Ausführung einzelner Zellenverwertungsschritte steuert, und
eine Haltevorrichtung (5) zum Halten der Probe, wobei die Haltevorrichtung enthält:
ein Gehäuse (59), das so abgedichtet werden kann, dass die Probe in einer definierten Atmosphäre gelagert/behandelt wird,
einen verschließbaren Einlass (51), der mit dem ersten Ventil (22) verbunden ist, und einen verschließbaren Auslass (52), wobei der Auslass (52) der Haltevorrichtung mit dem zweiten Ventil (42) verbunden ist, und
eine Befestigungsvorrichtung (57) an dem Einlass (51) der Haltevorrichtung und an dem Auslass (52) der Haltevorrichtung zum lösbaren Befestigen des Endes beziehungsweise der Enden der Probe.

2. Vorrichtung nach Anspruch 1, wobei die Anzahl der Ventile (22, 42) der Anzahl der Abgabebehälter (2) entspricht und wobei jedes der ersten Ventile (22) mit dem Auslass (21) eines Abgabebehälters verbunden ist und wobei jeder Abgabebehälter (2) mit einem der ersten Ventile (22) verbunden ist.

3. Vorrichtung nach Anspruch 2, die ferner eine erste Fluidführungsvorrichtung (6) mit Einlässen (61) und mit einem Auslass (62) enthält, wobei jeder Einlass (61) der ersten Fluidführungsvorrichtung (6) mit einem der ersten Ventile (22) verbunden ist, wobei der Einlass (51) der Haltevorrichtung mit dem Auslass (62) der ersten Fluidführungsvorrichtung verbunden ist.

4. Vorrichtung (1) nach einem der vorhergehenden Ansprüche, die ferner eine zweite Fluidführungsvorrichtung (7) mit einem Einlass (71) und mit Auslässen (72) enthält, wobei jeder der Auslässe (72) der zweiten Fluidführungsvornchtung mit dem Einlass (41) eines Sammelbehälters verbunden ist, wobei der Einlass (71) der zweiten Fluidführungsvorrichtung mit dem Auslass (52) der Haltevorrichtung verbunden ist.

5. Vorrichtung nach einem der vorhergehenden Ansprüche, wobei die Haltevorrichtung eine Länge (56) aufweist, die an die Größe der Probe angepasst werden kann.

6. Vorrichtung nach einem der vorhergehenden Ansprüche, wobei die Vorrichtung ferner eine Heizeinrichtung (58) zum Beheizen von Teilen der Vorrichtung enthält.

7. Vorrichtung nach einem der vorhergehenden Ansprüche, wobei die Vorrichtung ferner eine Luftfeuchteeinrichtung enthält, um die Feuchtigkeit innerhalb des Gehäuses (59) zu ermöglichen und/oder zu steuern, und/oder eine Begasungseinrichtung enthält, um die Gaszusammensetzung innerhalb des Gehäuses (59) bereitzustellen und/oder zu steuern.

8. Vorrichtung nach einem der vorhergehenden Ansprüche, die ferner einen Sensor (8) zum Messen eines Betriebsparameters enthält, wobei der gemessene Betriebsparameter an eine Steuereinheit (9) übertragen wird, um den Betriebsparameter zu steuern und zu regeln, wobei der wenigstens eine Betriebsparameter aus einer Gruppe ausgewählt ist, bestehend aus Temperatur, Strömungsparameter, Druck, Begasung, Feuchtigkeit.

9. Verfahren nach einem der vorhergehenden Ansprüche, wobei die fluiden Medien aus einer Gruppe ausgewählt sind, bestehend aus Spülmedium, Stoppmedium, Enzymmedium und Inkubationsmedium.

10. Verfahren zum automatisierten Verwerten von Zellen einer Probe, wobei das Verfahren die folgenden Schritte umfasst:
Bereitstellen einer Probe,
Montieren der Probe in der Haltevorrichtung (5) der Vorrichtung (1) eines der vorhergehenden Ansprüche, und
Isolieren von Zellen aus der Probe durch Ausführen eines Spülschritts mit Medien unter Verwendung der Medienförderanordnung (3),
wobei die Steuereinheit (9) zumindest das zweite Ventil (42) in Abhängigkeit von einem Sensorsignal automatisch steuert.

11. Verfahren nach Anspruch 10, wobei der Isolierschritt eine Vielzahl von Schritten umfasst, die die fluiden Medien aus den Abgabebehältern durch die Probe hindurch fördern, wobei die Einstellung der Ventile (22, 42), der Einlässe (41, 51, 61, 71) und der Auslässe (21, 52, 62, 72) so gewählt wird, dass ein Medienfluid nicht mit einem anderen Medienfluid gemischt wird, und/oder wobei die Medien Kügelchen oder Fängerpartikel, die eine Beschichtung mit Target-spezifischen Molekülen enthalten, aufweisen.

12. Verfahren nach Anspruch 10 oder 11, wobei eine Steuereinheit (9) ferner zumindest eine Einrichtung, die aus der Gruppe ausgewählt wird, bestehend aus Medienförderanordnung (3), erstem Ventil (22), Heizeinrichtung (58) oder aus allen davon, in Abhängigkeit von dem Sensorsignal automatisch steuert, und/oder wobei der Sensor im Fall einer Abweichung eines Betriebsparameters innerhalb des Verfahrens einen Alarm erzeugt.

13. Verfahren nach einem der Ansprüche 10 bis 12, wobei die Haltevorrichtung (5) der Vorrichtung (1) nach einem der vorhergehenden Ansprüche auf der Einlassseite (551, 552, 553), auf der Auslassseite (521, 522, 523) und bei der Befestigungsvorrichtung (571, 572, 573) zumindest drei Verbinder umfasst, wobei die Probe vorzugsweise wenigstens drei Gefäße umfasst und an den wenigstens drei Verbindern der Befestigungsvorrichtung (571, 572, 573) der Haltevorrichtung (5) der Vorrichtung (1) nach einem der vorhergehenden Ansprüche montiert wird.

14. Verfahren nach einem der Ansprüche 10 bis 13, das ferner die Schritte Füllen der Probe nach der vaskularen Zellenisolation mit einer Färbelösung, Entnehmen der wenigstens drei Gefäße der Probe und Isolieren von Stammzellen der Probe enthält.

## Revendications

1. Appareil (1) pour une exploitation automatisée de cellules provenant d'un échantillon, comprenant :
une pluralité de récipients de distribution (2) pour le stockage séparé de milieux fluidiques, chaque récipient de distribution comprenant une sortie (21), dans lequel un agencement de fourniture de milieu (3) est associé à chacun des récipients de distribution (2) et une première soupape (22) est reliée à chacune des sorties (21),
une pluralité de récipients de collecte (4) pour collecter les milieux fluidiques, chaque récipient de collecte (4) comprenant une entrée (41), dans lequel une seconde soupape (42) est reliée à l'entrée (41) de chaque récipient de collecte,
une unité de commande (9) commandant au moins la seconde soupape (42) pour l'exécution d'étapes individuelles d'exploitation de cellules, et
un dispositif de montage (5) pour monter l'échantillon, le dispositif de montage comprenant :
un boîtier (59) pouvant être fermé hermétiquement de sorte que l'échantillon est stocké/traité dans une atmosphère définie,
une entrée pouvant être fermée (51) étant reliée à la première soupape (22), et une sortie pouvant être fermée (52), dans lequel la sortie (52) du dispositif de montage est reliée à la seconde soupape (42), et
un appareil de fixation (57) sur l'entrée (51) du dispositif de montage et la sortie (52) du dispositif de montage pour fixer de façon libérable la/les extrémité(s) de l'échantillon.

2. Appareil selon la revendication 1, dans lequel le nombre de soupapes (22, 42) correspond au nombre de récipients de distribution (2) et chacune des premières soupapes (22) est reliée à la sortie (21) d'un récipient de distribution et chaque récipient de distribution (2) est relié à l'une des premières soupapes (22).

3. Appareil selon la revendication 2, comprenant en outre un premier dispositif de guidage de fluide (6) avec des entrées (61) et une sortie (62), dans lequel chaque entrée (61) du premier dispositif de guidage de fluide (6) est reliée à l'une des premières soupapes (22), dans lequel l'entrée (51) du dispositif de montage est reliée à la sortie (62) du premier dispositif de guidage de fluide.

4. Appareil (1) selon l'une quelconque des revendications précédentes, comprenant en outre un second dispositif de guidage de fluide (7) avec une entrée (71) et des sorties (72), dans lequel chacune des sorties (72) du second dispositif de guidage de fluide est reliée à l'entrée (41) d'un récipient de collecte, dans lequel l'entrée (71) du second dispositif de guidage de fluide est reliée à la sortie (52) du dispositif de montage.

5. Appareil selon l'une quelconque des revendications précédentes, dans lequel le dispositif de montage comprend une extension longitudinale (56) pouvant être adaptée à la dimension de l'échantillon.

6. Appareil selon l'une quelconque des revendications précédentes, l'appareil comprenant en outre un dispositif de chauffage (58) pour chauffer des pièces de l'appareil.

7. Appareil selon l'une quelconque des revendications précédentes, dans lequel l'appareil comprend en outre un dispositif d'humidité pour permettre et/ou contrôler l'humidité à l'intérieur du boîtier (59) et/ou un dispositif de gazage pour fournir et/ou contrôler la composition gazeuse à l'intérieur du boîtier (59).

8. Appareil selon l'une quelconque des revendications précédentes, comprenant en outre un capteur (8) pour mesurer un paramètre de fonctionnement, dans lequel le paramètre de fonctionnement mesuré est transféré à une unité de commande (9) pour commander et réguler le paramètre de fonctionnement, de préférence dans lequel l'au moins un paramètre de fonctionnement est sélectionné parmi un groupe consistant en la température, le paramètre d'écoulement, la pression, le gazage, l'humidité.

9. Appareil selon l'une quelconque des revendications précédentes, dans lequel le milieu fluidique est sélectionné parmi un groupe consistant en un milieu de lavage, un milieu d'arrêt, un milieu enzymatique et un milieu d'incubation.

10. Procédé pour l'exploitation automatisée de cellules provenant d'un échantillon comprenant les étapes de
fournir un échantillon,
monter l'échantillon dans le dispositif de montage (5) de l'appareil (1) selon l'une quelconque des revendications précédentes et
isoler des cellules provenant de l'échantillon en exécutant une étape de lavage avec un milieu en utilisant l'agencement de fourniture de milieu (3)
dans lequel l'unité de commande (9) commande automatiquement au moins la seconde soupape (42) en fonction d'un signal de capteur.

11. Procédé selon la revendication 10, dans lequel l'étape d'isolement comprend une pluralité d'étapes transférant le milieu fluidique à partir des récipients de distribution à travers l'échantillon, dans lequel le réglage des soupapes (22, 42), des entrées (41, 51, 61, 71) et des sorties (21, 52, 62, 72) est sélectionné de façon à ce qu'un milieu fluidique ne soit pas mélangé à un autre milieu fluidique, et/ou dans lequel le milieu comprend des billes ou des particules de captation contenant un revêtement avec des molécules spécifiques à la cible.

12. Procédé selon la revendication 10 ou 11, dans lequel une unité de commande (9) commande en outre automatiquement au moins un dispositif sélectionné parmi le groupe consistant en l'agencement de fourniture de milieu (3), la première soupape (22), le dispositif de chauffage (58) ou tous ceux-ci en fonction du signal du capteur, et/ou dans lequel le capteur déclenche une alerte en cas d'écart d'un paramètre de fonctionnement dans le procédé.

13. Procédé selon l'une quelconque des revendications 10 à 12, dans lequel le dispositif de montage (5) de l'appareil (1) selon l'une quelconque des revendications précédentes comprend au moins trois connecteurs sur le côté entrée (551, 552, 553), le côté sortie (521, 522, 523) et sur l'appareil de fixation (571, 572, 573), dans lequel l'échantillon comprend de préférence au moins trois cuvettes et est monté sur les au moins trois connecteurs de l'appareil de fixation (571, 572, 573) du dispositif de montage (5) de l'appareil (1) selon l'une quelconque des revendications précédentes.

14. Procédé selon l'une quelconque des revendications 10 à 13, comprenant en outre les étapes de remplissage de l'échantillon après l'isolement de cellules vasculaires avec une solution de coloration, de retrait des au moins trois cuvettes de l'échantillon et d'isolement des cellules souches provenant de l'échantillon.
